(19) Europäisches Patentamt
European Patent Office
Office européen des brevets

(11) EP 3 173 083 B1

(12) **EUROPEAN PATENT SPECIFICATION**

(45) Date of publication and mention of the grant of the patent:
**05.07.2023 Bulletin 2023/27**

(21) Application number: **15203153.0**

(22) Date of filing: **30.12.2015**

(51) International Patent Classification (IPC):
***A61K 31/444*** $^{(2006.01)}$ ***A61P 29/00*** $^{(2006.01)}$
***A61P 9/10*** $^{(2006.01)}$ ***A61P 19/02*** $^{(2006.01)}$
***A61P 27/02*** $^{(2006.01)}$ ***A61K 33/24*** $^{(2019.01)}$
***A61K 31/555*** $^{(2006.01)}$

(52) Cooperative Patent Classification (CPC):
**A61K 31/555; A61P 9/10; A61P 19/02; A61P 27/02; A61P 29/00**

(54) **IN VIVO ANTIANGIOGENIC EFFECT OF A PALLADIUM COMPLEX**

IN-VIVO-ANTIANGIOGENE WIRKUNG EINES PALLADIUM-KOMPLEXES

EFFET ANTI-ANGIOGÉNIQUE IN VIVO D’UN COMPLEXE DE PALLADIUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2015 TR 201515168**

(43) Date of publication of application:
**31.05.2017 Bulletin 2017/22**

(73) Proprietor: **Istanbul Universitesi Rektorlugu
34452 Istanbul (TR)**

(72) Inventors:
- **Ilkay Armutak, Elif
34320 Istanbul (TR)**
- **Gürel Gürevin, Ebru
34134 Istanbul (TR)**
- **Kiyan, Hülya Tuba
26470 Eskisehir (TR)**
- **Ulukaya, Engin
Bursa (TR)**
- **Yilmaz, Veysel Turan
Bursa (TR)**

(74) Representative: **Mutlu, Aydin
Invokat Intellectual Property Services Ltd.
Kartaltepe Mh. Yildiztepe Sk.
No:6-Bakirköy
34145 Istanbul (TR)**

(56) References cited:
- **ENGIN ULUKAYA ET AL: "Anti-cancer activity of a novel palladium(II) complex on human breast cancer cellsand", EUROPEAN JOURNAL OF MEDICINAL CHEMISTRY, EDITIONS SCIENTIFIQUE ELSEVIER, PARIS, FR, vol. 46, no. 10, 29 July 2011 (2011-07-29) , pages 4957-4963, XP028390950, ISSN: 0223-5234, DOI: 10.1016/J.EJMECH.2011.07.055 [retrieved on 2011-08-05]**
- **ENGIN ULUKAYA ET AL: "Cell death-inducing effect of novel palladium(II) and platinum(II) complexes on non-small cell lung cancer cells in vitro", JOURNAL OF CANCER RESEARCH AND CLINICAL ONCOLOGY, SPRINGER, BERLIN, DE, vol. 137, no. 10, 2 August 2011 (2011-08-02), pages 1425-1434, XP019951973, ISSN: 1432-1335, DOI: 10.1007/S00432-011-1021-1**
- **ZELAL ADIGUZEL ET AL: "Biochemical and Proteomic Analysis of a Potential Anticancer Agent: Palladium(II) Saccharinate Complex of Terpyridine Acting through Double Strand Break Formation", JOURNAL OF PROTEOME RESEARCH., vol. 13, no. 11, 7 November 2014 (2014-11-07), pages 5240-5249, XP055360250, US ISSN: 1535-3893, DOI: 10.1021/pr5006718**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 173 083 B1

## Description

The present invention is related to antiangiogenic effect of a Palladium complex [Pd(sac)(terpy)] (sac)•4H20 (sac=saccharinate and terpy=2,2':6',2"-terpyridine)]. In another aspect, the present invention is related to said Palladium complex for use in the treatment of diseases in which angiogenesis is effective.

## Technical Field of the Invention

Angiogenesis which refers to formation of new blood vessels from pre-existing blood vessels, is physiologically active in development of embryo and healing the wounds. Chronic inflammation such as rheumatoid arthritis, cardiovascular diseases such as psoriasis and atherosclerosis and diabetic retinopathy have an important place in the pathogenesis of ocular diseases *(*Carmeliet, P., Jain, R.K., 2000. Angiogenesis in cancer and other diseases. Nature, 407(6801): 249-257*;* Carmeliet, P., 2005. Angiogenesis in life, disease and medicine. Nature, 438(7070): 932-936). It also plays a role in the development and metastasis of various types of cancer. Antiangiogenic approach is promising hope in prevention and treatment for same pathologies and some tumors *(*Griffioen, A. W, Molema, G., 2000. Angiogenesis: potentials for pharmacologic intervention in the treatment of cancer, cardiovascular diseases, and chronic inflammation. Pharmacol. Rev., 52(2): 237-268*;* Carmeliet, P., 2003. Angiogenesis in health and disease. Nat. Med., 9(6): 653-660*;* Ferrara, N., Kerbel, R.S., 2005. Angiogenesis as a therapeutic target. Nature, 438(7070): 967-974*;* Eichhorn, M.E., Kleespies, A., Angele, M.K., Jauch, K.W., Bruns, C.J., 2007. Angiogenesis in cancer: molecular mechanisms, clinical impact. Langenbecks Arch. Surg., 392(3): 371-379*;* Ribatti, D., 2009. History of Research on Tumour Angiogenesis. Springer, Italy, pp. 1-17*).* Antiangiogenesis approach which plays a key role for treatment of the primary tumours and the prevention for metastasis is first brought forward in 1971 by Judah Folkman *(*Ribatti, D., 2010. The inefficacy of antiangiogenic therapies. J. Angiogenes. Res., 2:27. doi: 10.1186/2040-2384-2-27*).* From past to present ongoing researches of antiangiogenesis provide many antiangiogenic drugs to take a part in the treatment of cancer.

Bevacizumab, is primarily discovered as a result of researches and still used in clinical treatment of colon cancer as an antiangiogenic drug *(*Bikfalvi, A., 2006. Angiogenesis: health and disease. Ann. Oncol., 17(10): 65-70*).*

The cytotoxic effect, the apoptotic effect and the antiproliferative effect of the palladium complex which is the subject of the invention are previously described. Such studies up to today *in vitro* conditions, are carried out either in different cancer cell lines or *in vivo.* However, no research explaining the antiangiogenic feature of said molecule or about this topic has not been found.

Palladium-like drugs with antiangiogenic properties are available in the prior art and one of them is cisplatin. Cisplatin, being an antineoplastic agent is an organic platinum complex. However, it has some side effects such as kidney failure, bone marrow suppression and acute infection. Moreover, oxaliplatin, as one of platinum-based drugs, also has antineoplastic properties, but this drug also has serious effects on the nervous system. Therefore, there is a need in the present technique for new molecules having antiangiogenic effect.

## Description of Figures

Figure 1, is a representative image that shows how to perform the CAM experiment.

Figure 2, is a representative stereomicroscope image that shows the very strong antiangiogenic effect of Cortisone on CAM.

Figure 3, is a representative stereomicroscope image that shows the strong antiangiogenic effect of Prednisone on CAM.

Figure 4, is a representative stereomicroscope image that shows the strong antiangiogenic effect of (±)-Thalidomide on CAM.

Figure 5, is a representative stereomicroscope image that shows irritant effect of sodium dodecyl sulphate on CAM.

Figure 6, is a representative stereomicroscope image that shows the very strong antiangiogenic effect of Palladium complex on CAM.

**Description of the Invention**

The present invention, is related to the provision of a Palladium complex for use as an antiangiogenic agent in the treatment of a disease selected from a group comprising chronic inflammation, cardiovascular diseases and ocular diseases developed with angiogenesis. The mentioned Palladium complex is named [Pd(sac)(terpy)] (sac)•4H20 (sac=saccharinate and terpy=2,2':6',2"-terpyridine)] and is shown by the following formula.

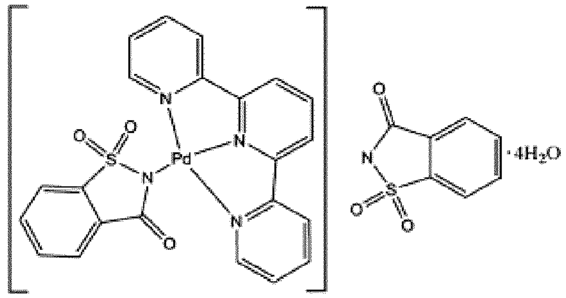

**Formula (I)-** Palladium complex of the invention

Within the scope of the invention, said chronic inflammation diseases can be rheumatoid arthritis and/or psoriasis. In a preferred embodiment of the invention, cardiovascular disease can select as atherosclerosis. In an embodiment of the invention, ocular disease can be selected as diabetic retinopathy.

In another aspect, the present invention is related to a pharmaceutical composition comprising a Palladium complex having formula (I) for use as an antiangiogenic agent in the treatment of a disease selected from a group comprising chronic inflammation, cardiovascular diseases and ocular diseases developed with angiogenesis, and at least one pharmaceutically acceptable carrier. Said pharmaceutical composition can be administered to the patient via oral or parenteral route.

In a study performed on the antiangiogenic effect of palladium complex of the invention, a method which is named chorion allantoic membrane (CAM) model is used. The model in question is a method developed by the embryologists more than 50 years ago, has a broad area of usage in embryonic researches *(*Auerbach, R., Lewis, R., Shinners, B., Kubai, L., Akhtar, N., 2003. Angiogenesis assays: a critical overview. Clin. Chem., 49(1): 32-40*)*. Since 1956, the model is used in cancer and metastasis researches. The model has been started to be used in angiogenesis studies by Folkman in 1976. CAM, is a non-embryo membrane which is formed by the merger of chorion and allantois as a result of an incubation of four-days. Although it is widely used in embryonic researches, it also has a broad range of usage in cancer and metastasis researches *(*Ribatti, D., 2010. The inefficacy of antiangiogenic therapies. J. Angiogenes. Res., 2:27. doi: 10.1186/2040-2384-2-27*)*. It is a method informing about antiangiogenic, anti-inflammatory, antirritant features of various chemicals, besides, it is also a method of which reveals the characteristics such as possible membrane irritation, decomposition, coagulation and toxicity. The method, is one of the most commonly used methods since it is quick, cheap and semi quantitative and enable to the scanning of many new agents *(*Cimpean, A.M., Ribatti, D., Raica, M., 2008. The chick embryo chorioallantoic membrane as a model to study tumor metastasis. Angiogenesis, 11(4): 311-319*;* Ribatti, D., 2008. Chick embryo chorioallantoic membrane as a useful tool to study angiogenesis. Int. Rev. Cell Mol. Biol., 270: 181-224*)*. Moreover, CAM model, besides the fact that it can easily and quickly evaluate the result of the experiments, is compatible with mammalian xenographs, suitable for surgical procedures, reliable and repeatable, it is superior method than the other methods due to the fact it is an alternative method for in vivo animal experiments which are used in the determination of the effect and the irritant profiles of chemical compounds especially cosmetics and which results in the death of many animals *(Özgürtaş, T., 2009. Anjiyogenezde bir in-vivo model: civciv koriyoallantoik membran. Gülhane Tıp Dergisi, 51,* 67-69).

The demonstration of the angiogenic effect of the Palladium complex according to the invention by using the CAM model is a first in this area. Accordingly, a new feature of the Palladium complex which is 'antiangiogenic effect' has been demonstrated with CAM model which is an *in vivo* method.

## EXAMPLES

### Preparation of CAM Experiments

The experiment Protocol which was reported in 1967 by D'Arcy and Howard and in 2002 by Bürgermeister and his colleagues was used (D'Arcy, P.F., Howard, E.M., 1967. A new anti-inflammatory test, utilizing the chorio-allantoic membrane of the chick embryo. Br. J. Pharmacol. Chemother., 29(3): 378-38*;* Bürgermeister, J., Paper, D.H., Vogl, H., Linhardt, R.J., Franz, G., 2002. LaPSvS1, a (1-->3)-beta-galactan sulfate and its effect on angiogenesis in vivo and in vitro. Carbohydr. Res., 337(16): 1459-1466*).*

### Preparation of Samples

The palladium complex, 5 mg/ml (50 μg /pellet), the standard control agents sodium dodecyl sulfate (SDS), cortisone, prednisone, (±)-thalidomide has been suspended in 2,5 % (w/v) agar at a final concentration of 50 μg /pellet by heating (60 ºC<). Then, 10 μl is taken from this suspension and provided to become frozen pellet at room temperature by being applied on stainless steel cylinder supports with a diameter 5 mm. Pellets are applied on the surface of CAM.

### Procedure for CAM Experiment

The procedure for CAM experiment is given in Figure 1 *(Kiyan, H.T., 2010. Bazı Hypericum Türlerinin Uçucu Yağ Bileşimleri ve Antianjiyojenik Aktiviteleri, Yüksek Lisans Tezi, Anadolu Üniversitesi, Sağlık Bilimleri Enstitüsü, Farmakognozi Programi, 19p).* Accordingly, fertilized chicken eggs to be used in experiment are incubated at 36,5ºC and in an incubator with 80% relative humidity (climatization cabin) at horizontal position for 72 hours before the experiment. In this period, eggs are carefully upturned time to time. At the end of the incubation period, firstly, from the bottom side of eggs with the help of the sterile injectors, slowly 10-15 ml of albumin (egg white) and then from the top side of (more upright and sharp part) eggs with the help of the forceps, shell and membrane are carefully removed (Figure-1, A). Living and developing embryo CAM immediately taken under protection with a stretch film, is waited again at 36,5 ºC and in an incubator with 80% relative humidity for another 72 hours (Figure-1, B). When diameter of the CAM is detected approximately 2 cm, one piece of freshly prepared sample pellet for each egg is placed capillaries on the CAM (Figure-1, C). 15 piece from each material is applied on CAM an equal number of eggs and covered with stretch film. Before the evaluations, eggs are incubated pellet placed in under specified conditions for another 24 hours. End of the period, influences of the material containing pellet on capillaries is evaluated by the help of stereomicroscope (Figure-1, D). Sodium dodecyl sulphate (SDS), cortisone, prednisone, and (±)-thalidomide as standard control materials, agar as a blind are used. Every week 15 eggs, a total of 45 eggs are used for each concentration of each substance tested.

### Evaluation of Results

A scoring system developed by Krenn and Paper (2009) is used for the evaluation of the results of control substances and samples on CAM (Krenn, L., Paper, D.H., 2009. Inhibition of angiogenesis and inflammation by an extract of red clover (Trifolium pratense L.). Phytomedicine, 16(12): 1083-1088). According to the system (Table 1), results are calculated based on scores obtained by applying the formula given below. Evaluation results are shown in Table 2.

**Table 1.** Values of Scores Used for the Evaluation of the Angiogenic Effect on CAM

| Score | Effect | Impression/Explanation |
|---|---|---|
| < 0.5 | None | Generation of normal embryo. |
| 0.5-0.75 | Weak | No veinless capillary area. Density of capillary decreased, but not much wider than pellet. |
| > 0.75-1 | Strong | Non-capillary area is low or density of capillary decreased in specific area. Effect, not more than twice the area of pellet. |
| >1 | Very Strong | There is a non-capillary area around the pellet at least 2 times of size. |

**Formula used for Evaluation**

$$\text{Average score} = \frac{\text{Number of eggs (Score 2) x 2 + Number of eggs (Score 1) x 1}}{\text{Total number of eggs (Score 0, 1, 2)}}$$

**CAM Activity Results**

Result of the evaluation performed according to scoring system in Table 1, is calculated according to the given formula and scores are shown in Table 2. Effect of palladium complex and standard control materials on CAM are viewed with stereomicroscope (Figure 2-6). Hereunder these results, cortisone (Figure 2), prednisone (Figure 3) and (±)-thalidomide (Figure 4) at 50 µg/pellet concentration compared with the positive standards, closed the cortisone (1.2 ± 0.2) and a score (1.1 ± 0.2) higher than (±)-thalidomide (0.9 ± 0.2) and prednisone (0.9 ± 0.7), show very strong antiangiogenic effect. Tested complex at 50 µg/pellet, show no toxic or irritant effect.

**Table 2.** CAM Activity Results

| **Test Materials** | **Score** | **Irritation (%)** | **Concentration (µg/pellet)** |
|---|---|---|---|
| Palladium complex | 1.1 ± 0.2 | - | 50 µg/pellet |
| Agar | 0.2 ± 0.2 | - | Blind (% 2.5, a/h) |
| Prednisone | 0.9 ± 0.7 | - | Positive control (50 µg/pellet) |
| Cortisone | 1.2 ± 0.2 | | Positive control (50 µg/pellet) |
| (±)-Thalidomide | 0.9 ± 0.2 | - | Positive control (50 µg/pellet) |
| Sodium dodecyl sulphate (SDS) | 0.1 ± 0.1 | 87 ± 5 | Negative control (50 µg/pellet) |

## Claims

1. A Palladium complex having Formula (I) for use as an antiangiogenic agent in the treatment of a disease selected from a group comprising chronic inflammation, cardiovascular diseases and ocular diseases developed with angiogenesis.

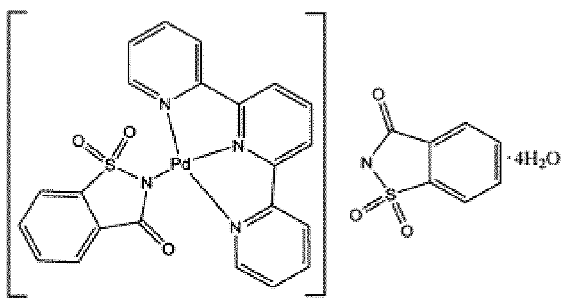

(I)

2. A Palladium complex for use according to claim 1, wherein the chronic inflammation disease is rheumatoid arthritis and/or psoriasis.

3. A Palladium complex for use according to claim 1, wherein the cardiovascular disease is atherosclerosis.

4. A Palladium complex for use according to claim 1, wherein the ocular disease is diabetic retinopathy.

5. A pharmaceutical composition comprising a Palladium complex having Formula (I) for use as an antiangiogenic agent in the treatment of a disease selected from a group comprising chronic inflammation, cardiovascular diseases and ocular diseases developed with angiogenesis, and at least one pharmaceutically acceptable carrier.

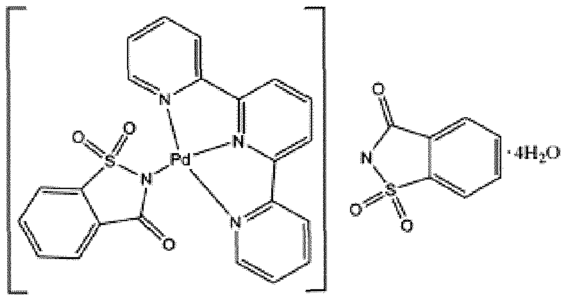

(I)

## Patentansprüche

1. Palladiumkomplex der Formel (I) zur Verwendung als antiangiogenes Mittel bei der Behandlung einer Erkrankung, die aus einer Gruppe ausgewählt ist, die chronische Entzündungen, kardiovaskuläre Erkrankungen und Augenerkrankungen, welche sich durch Angiogenese entwickeln, umfasst.

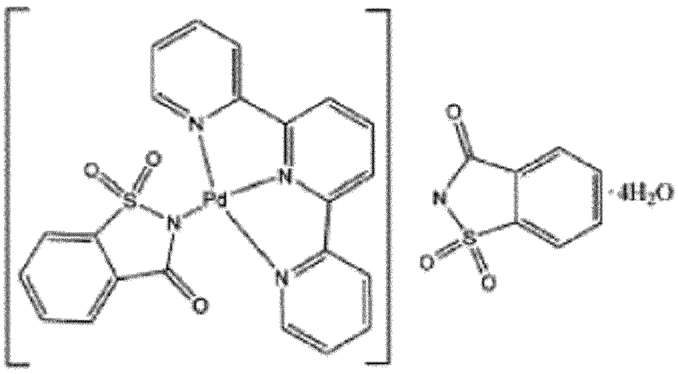

(I)

2. Palladiumkomplex zur Verwendung gemäß Anspruch 1, wobei die chronischentzündliche Erkrankung rheumatoide Arthritis und/oder Psoriasis ist.

3. Palladiumkomplex zur Verwendung gemäß Anspruch 1, wobei die kardiovaskuläre Erkrankung Atherosklerose ist.

4. Palladiumkomplex zur Verwendung gemäß Anspruch 1, wobei die Augenerkrankung diabetische Retinopathie ist.

5. Pharmazeutische Zusammensetzung, die einen Palladiumkomplex der Formel (I) und mindestens einen pharmazeutisch verträglichen Träger umfasst, zur Verwendung als antiangiogenes Mittel bei der Behandlung einer Erkrankung, die aus einer Gruppe ausgewählt ist, die chronische Entzündungen, kardiovaskuläre Erkrankungen und Augenerkrankungen, welche sich durch Angiogenese entwickeln, umfasst.

## Revendications

1. Complexe de palladium de formule (I) pour utilisation en tant qu’agent anti-angiogénique dans le traitement d’une maladie choisie dans l’ensemble comprenant les maladies inflammatoires chroniques, maladies cardiovasculaires et maladies oculaires qui s’accompagnent d’une angiogenèse.

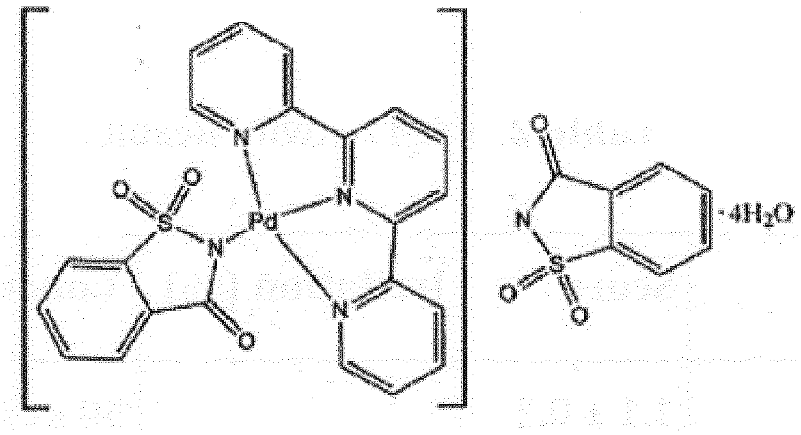

(I)

2. Complexe de palladium pour utilisation conforme à la revendication 1, pour lequel la maladie inflammatoire chronique est une polyarthrite rhumatoïde et/ou un psoriasis.

3. Complexe de palladium pour utilisation conforme à la revendication 1, pour lequel la maladie cardiovasculaire est une athérosclérose.

4. Complexe de palladium pour utilisation conforme à la revendication 1, pour lequel la maladie oculaire est une rétinopathie diabétique.

5. Composition pharmaceutique comprenant un complexe de palladium de formule (I) pour utilisation en tant qu'agent anti-angiogénique dans le traitement d'une maladie choisie dans l'ensemble comprenant les maladies inflammatoires chroniques, maladies cardiovasculaires et maladies oculaires qui s'accompagnent d'une angiogenèse, et au moins un véhicule pharmacologiquement admissible.

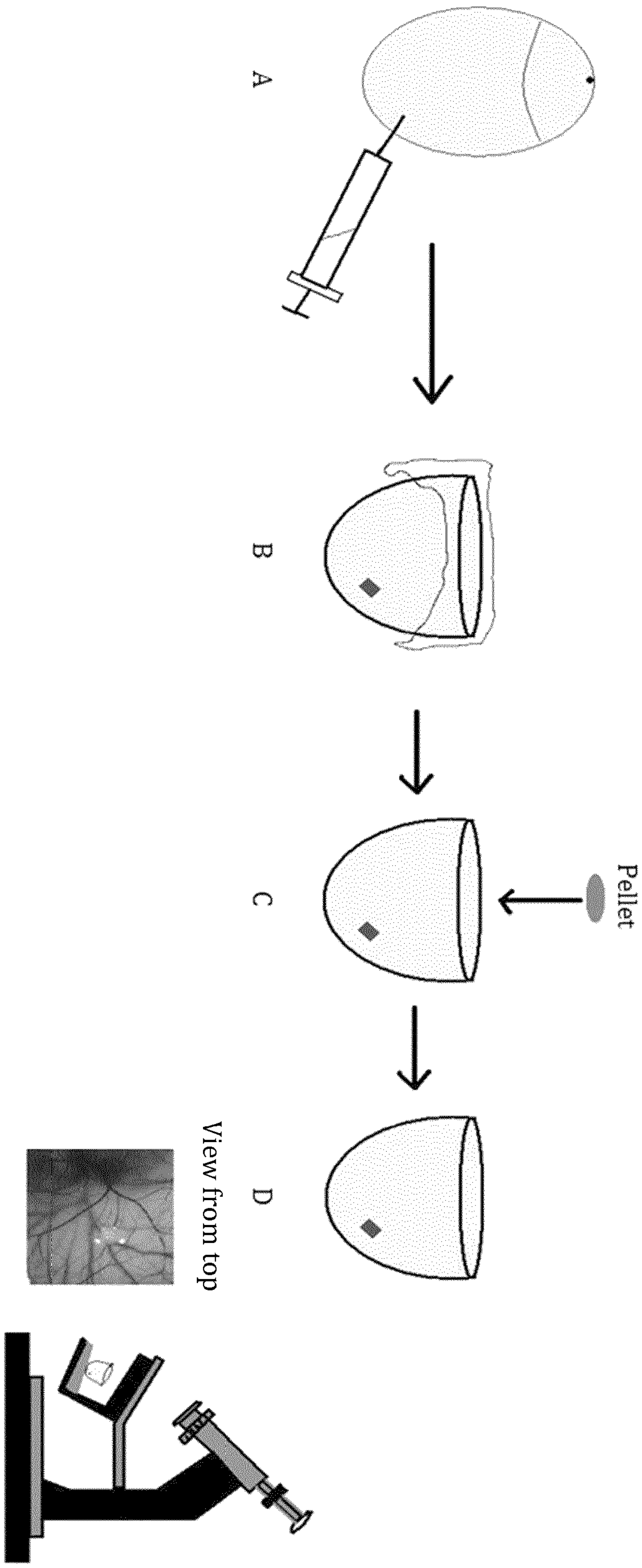


**Figure - 1**

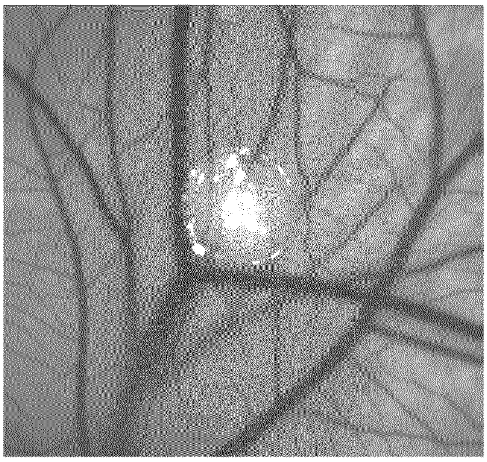

Figure - 2

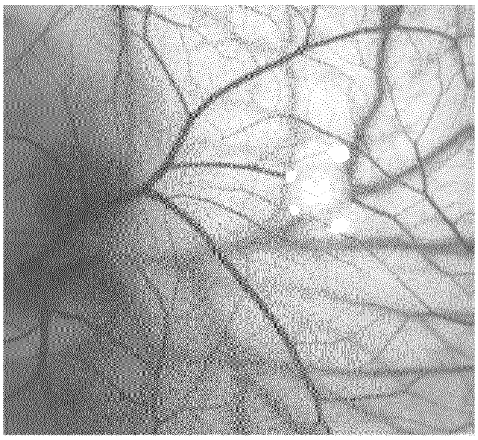

Figure - 3

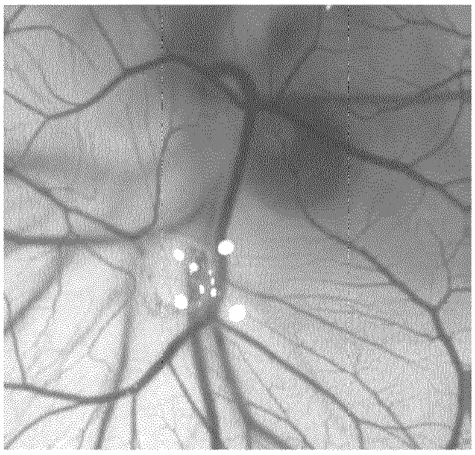

Figure - 4

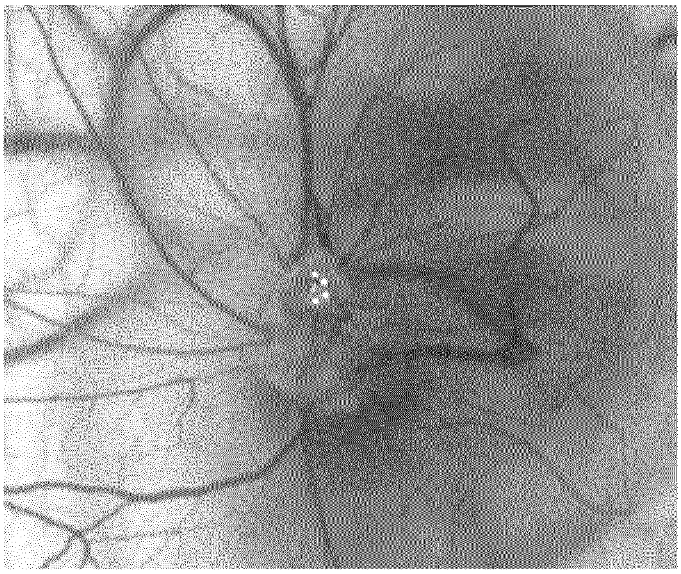

Figure - 5

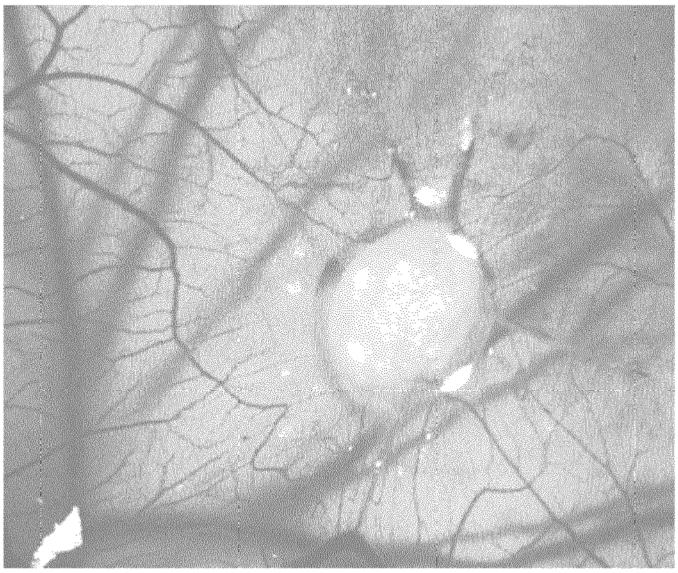

Figure - 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CARMELIET, P. ; JAIN, R.K.** Angiogenesis in cancer and other diseases. *Nature,* 2000, vol. 407 (6801), 249-257
- **CARMELIET, P.** Angiogenesis in life, disease and medicine. *Nature,* 2005, vol. 438 (7070), 932-936
- **GRIFFIOEN, A. W ; MOLEMA, G.** Angiogenesis: potentials for pharmacologic intervention in the treatment of cancer, cardiovascular diseases, and chronic inflammation. *Pharmacol. Rev.,* 2000, vol. 52 (2), 237-268
- **CARMELIET, P.,.** Angiogenesis in health and disease. *Nat. Med.,* 2003, vol. 9 (6), 653-660
- **FERRARA, N. ; KERBEL, R.S.,.** Angiogenesis as a therapeutic target. *Nature,* 2005, vol. 438 (7070), 967-974
- **EICHHORN, M.E., ; KLEESPIES, A. ; ANGELE, M.K. ; JAUCH, K.W. ; BRUNS, C.J.** Angiogenesis in cancer: molecular mechanisms, clinical impact. *Langenbecks Arch. Surg.,* 2007, vol. 392 (3), 371-379
- **RIBATTI, D.** History of Research on Tumour Angiogenesis. Springer, 2009, 1-17
- **RIBATTI, D.,.** The inefficacy of antiangiogenic therapies. *J. Angiogenes. Res.,* 2010, vol. 2 (27
- **BIKFALVI, A.** Angiogenesis: health and disease. *Ann. Oncol.,* 2006, vol. 17 (10), 65-70
- **AUERBACH, R. ; LEWIS, R. ; SHINNERS, B. ; KUBAI, L., ; AKHTAR, N.** Angiogenesis assays: a critical overview. *Clin. Chem.,* 2003, vol. 49 (1), 32-40
- **RIBATTI, D.,.** The inefficacy of antiangiogenic therapies. *J. Angiogenes. Res.,* 2010, vol. 2 (27
- **CIMPEAN, A.M. ; RIBATTI, D. ; RAICA, M.** The chick embryo chorioallantoic membrane as a model to study tumor metastasis. *Angiogenesis,* 2008, vol. 11 (4), 311-319
- **RIBATTI, D.** Chick embryo chorioallantoic membrane as a useful tool to study angiogenesis. *Int. Rev. Cell Mol. Biol.,* 2008, vol. 270, 181-224
- **D'ARCY, P.F. ; HOWARD, E.M.,.** A new anti-inflammatory test, utilizing the chorio-allantoic membrane of the chick embryo. *Br. J. Pharmacol. Chemother.,* 1967, vol. 29 (3), 378-38
- **BÜRGERMEISTER, J. ; PAPER, D.H., ; VOGL, H. ; LINHARDT, R.J. ; FRANZ, G.** aPSvS1, a (1-->3)-beta-galactan sulfate and its effect on angiogenesis in vivo and in vitro. *Carbohydr. Res.,* 2002, vol. 337 (16), 1459-1466
- **KRENN, L. ; PAPER, D.H.** Inhibition of angiogenesis and inflammation by an extract of red clover (Trifolium pratense L.). *Phytomedicine,* 2009, vol. 16 (12), 1083-1088